# EUROPÄISCHE PATENTSCHRIFT

(11) **EP 3 030 886 B1**
(45) Veröffentlichungstag und Bekanntmachung des Hinweises auf die Patenterteilung: **22.09.2021**
(21) Anmeldenummer: 14761698.1
(22) Anmeldetag: 01.08.2014
(51) Int. Cl.: G01N 21/64, G01N 31/22, G01N 21/78

(54) **OPTISCHER SENSOR UND MESSANORDNUNG ZUM QUANTITATIVEN NACHWEIS EINES ANALYTEN IN EINER PROBE**
OPTICAL SENSOR AND ARRANGEMENT FOR QUANTITATIVELY DETECTING AN ANALYTE
CAPTEUR OPTIQUE ET ARRANGEMENT POUR LA DÉTECTION QUANTITATIVE D'UN ANALYTE

(30) Priorität: 09.08.2013 DE 102013108659
(43) Veröffentlichungstag der Anmeldung: 15.06.2016
(73) Patentinhaber: PreSens Precision Sensing GmbH, 93053 Regensburg (DE)
(72) Erfinder: RIECHERS, Daniel, 93051 Regensburg (DE); APOSTOLIDIS, Athanasios, 93055 Regensburg (DE)
(74) Vertreter: Reichert & Lindner Partnerschaft Patentanwälte
(86) Internationale Anmeldenummer: PCT/IB2014/063620
(87) Internationale Veröffentlichungsnummer: WO 2015/019260

(56) Entgegenhaltungen:
- WO-A2-97/19348
- WO-A2-2004/059281
- US-A- 4 557 900
- US-A- 5 403 746
- US-B1- 6 428 748
- None

## Beschreibung

Die Erfindung betrifft einen Sensor zum quantitativen Nachweis eines Analyten in einer Probe, bei dem ein optisches Verhalten wenigstens eines Farbstoffs zum quantitativen Nachweis des Analyten genutzt wird.

Die Übersetzung DE69430003T2 der europäischen Patentschrift EP 0 731 664 B1 zur internationalen Anmeldung PCT/US94/12146 offenbart einen Sensor mit einem in einer wässrigen Phase enthaltenen Indikatorfarbstoff. Der Farbstoff kann sich in einer an Teilchen ad- oder in Teilchen absorbierten Flüssigkeit befinden, die Teilchen ihrerseits sind dabei in ein gas- und lichtdurchlässiges polymeres Material eingebettet, welches kein flüssiges Wasser durchlässt. Die wässrige Phase kann auch in Mikrokammern eingeschlossen sein. Ferner kann die wässrige Phase einen Puffer enthalten sowie Salze zur Einstellung einer gewünschten Osmolarität.

Die internationale Veröffentlichung WO 00/26655 A1 der internationalen Anmeldung PCT/US99/25506 offenbart einen Farbstoff, der in einer Vertiefung einer Sensormembran eingeschlossen ist. Eine perforierte Metallscheibe ist unter der Sensormembran angeordnet, um ein Anschwellen der Farbstoffschicht zu verhindern.

Die deutsche Übersetzung DE 69219061 T2 der europäischen Patentschrift EP 0 539 175 B1 beschreibt unter anderem die Imprägnierung eines Trägers mit einer Reagenzzusammensetzung. Diese enthält einen Puffer und kann als Bindemittel z.B. Cellulose, Gummi arabicum oder Polyvinylpyrrolidon enthalten.

Die deutsche Übersetzung DE 69612017 T2 der europäischen Patentschrift EP 0 873 517 B1 zur internationalen Anmeldung PCT/US96/16469 beschreibt eine wässrige Phase mit Indikator und Puffer, welche sich in einer zweiten hydrophoben Phase befindet. Dabei kann diese Emulsion Feuchthaltemittel enthalten. Die wässrige Phase befindet sich in Mikrokompartimenten. Es können Substanzen zugegeben werden, welche den osmotischen Druck regeln.

Die deutsche Offenlegungsschrift DE 2 134 910 betrifft einen Farbstoff zum Anfärben weißer Blutzellen sowie ein Verfahren zur analytischen Bestimmung von weißen Blutzellen im Blut. Eine wässrige Lösung mit einem Farbstoff wird mit einer Blutprobe versetzt. Dabei enthält die wässrige Lösung Zusatzstoffe, um einen pH-Wert und eine Osmolalität innerhalb der normalen Bereiche für menschliches Blutplasma zu halten.

Die internationale Veröffentlichung WO 2009/140559 A1 zur internationalen Anmeldung PCT/US2009/044048 offenbart ein Sensorelement mit Schichtstruktur. Ein Indikator ist dabei an eine poröse Stützmembran gebunden, welche auf einem Polymersubstrat angeordnet ist. Die Stützmembran kann auch aus Plastikgewebe bestehen.

Die US-Veröffentlichung US 2004/171094A1 betrifft einen in hydrophobe Polymerteilchen eingeschlossenen Farbstoff mit analytabhängiger Phosphoreszenz. Die Teilchen können ihrerseits in eine Schicht oder Matrix eingebettet sein, welche wasseraufnehmend sein und bei Wasseraufnahme anschwellen kann.

Die US-Veröffentlichung US 2008/215254 A1 beschreibt Sensoren, welche aus einer oder mehreren auf einem transparenten Träger angeordneten Schichten bestehen. Die Schichten können aus einem Polymer bestehen und beispielsweise in den Kavitäten einer Mikrotiterplatte oder am Ende eines Lichtleiters angeordnet werden.

Das US-Patent US 4,557,900 betrifft einen optischen Sensor zum Nachweis eines in einer Flüssigkeit gelösten Gases. In einer für das Gas durchlässigen, für die Flüssigkeit undurchlässigen Matrix sind Partikel verteilt, welche mit einem optischen Indikator versehen sind. Der Indikator spricht auf das Gas an.

Das US-Patent US 6,428,748 B1 offenbart einen Detektor zum Nachweis eines Analyten in einer Probe. In einem Träger ist ein Sensorgemisch eingeschlossen, welches einen Indikatorfarbstoff, einen Puffer und eine Substanz zur Beeinflussung der Osmose enthält. Diese Substanz wird dem Sensorgemisch zugesetzt, um einem Abströmen von Wasser aus dem Sensor in die Probe entgegenzuwirken. Dabei kann eine Osmolarität eingestellt werden, welche über der Osmolarität einer gegebenen Probe liegt.

Die internationale Veröffentlichung WO 2004/059281 A2 betrifft einen Sensor zum Nachweis eines Analyten für den Einsatz bei Verpackungen. Der Detektor hat eine Schichtstruktur. Der Detektionsmechanismus beruht unter anderem auf Antikörpern in Verbindung mit fluoreszierenden Farbstoffen oder elektrisch leitfähigen Substanzen.

Optische Sensoren zur Detektion saurer oder basischer Gase, zum Beispiel Ammoniak oder Schwefeldioxid, in gasförmigen Proben oder in Flüssigkeiten gelöst, sind hinlänglich bekannt. Diese Sensoren verfügen über einen Farbstoff mit einem optischen Verhalten, das durch das nachzuweisende Gas beeinflusst wird, oftmals indirekt, etwa über eine Änderung des pH-Werts einer den Farbstoff enthaltenden Pufferlösung. In der Regel ist die Pufferlösung mit dem Farbstoff von der zu untersuchenden Probe durch ein gaspermeables Material, zum Beispiel ein gaspermeables Polymer, getrennt. Gelangt nun Wasser aus der Probe in die Pufferlösung, weil das gaspermeable Material selbst wasseraufnahmefähig ist und Wasser dann aus dem gaspermeablen Material in die Pufferlösung gelangt, oder weil Wasser in Form von Dampf durch das gaspermeable Material in die Pufferlösung diffundiert, so ändert sich die Konzentration des Puffers und damit der pH-Wert. Dadurch ergeben sich Bedingungen im Sensor, welche nicht mehr der Kalibrierung des Sensors entsprechen. Da der Wassertransport in die Pufferlösung von den Osmolalitäten der Pufferlösung und der Probe bestimmt wird, resultiert eine Osmolalitätsquerempfindlichkeit des Sensors. Diese ist vor allem dann problematisch, wenn sich die Osmolalität der Probe über die Zeitdauer einer Messung ändert. Diese Problematik tritt beispielsweise im Biotechnologie-Bereich auf, hier werden Sensoren der beschriebenen Art zur Überwachung von Bioprozessen, etwa Fermentationen oder Zellkultivierungen eingesetzt. Auch im medizinischen Bereich, bei online oder offline Messungen in Blut, Urin oder Geweben, können starke Änderungen der Osmolalität auftreten, welche bei den beschriebenen Sensoren nach dem Stand der Technik zu großen Messfehlern führen.

Aufgabe der Erfindung ist es, einen Sensor bereitzustellen, der eine gegenüber dem Stand der Technik verringerte Osmolalitätsquerempfindlichkeit aufweist, und dessen Kalibrierung somit von der Osmolalität der Probe weitgehend unabhängig ist.

Diese Aufgabe wird gelöst durch einen Sensor gemäß Anspruch 1.

Der erfindungsgemäße Sensor zum quantitativen Nachweis eines Analyten in einer Probe umfasst wenigstens einen Farbstoff, der ein optisches Verhalten aufweist, welches innerhalb des Sensors durch den Analyten beeinflussbar ist, welches also direkt oder, bedingt durch die Ausgestaltung des Sensors, indirekt von dem Analyten abhängt. Dies bedeutet, aus dem optischen Verhalten des wenigstens einen Farbstoffs kann quantitativ auf den Analyten geschlossen werden, es kann also beispielsweise eine Konzentration oder ein Partialdruck für den Analyten ermittelt werden. Hierzu wird vorteilhaft eine Kalibrierung des Sensors verwendet. Unter quantitativem Nachweis des Analyten wird verstanden, dass ein Wert für die Konzentration oder den Partialdruck des Analyten bis auf fachübliche Fehlergrenzen bestimmt wird, aber auch, dass lediglich festgestellt wird, dass die Konzentration oder der Partialdruck des Analyten innerhalb eines vorgegebenen Bereichs liegen; der vorgegebene Bereich kann dabei eine Untergrenze und eine Obergrenze, oder nur entweder eine Untergrenze oder eine Obergrenze haben.

Ferner umfasst der erfindungsgemäße Sensor ein Medium, in welchem der wenigstens eine Farbstoff enthalten ist, sowie ein Restriktionsmittel, durch welches eine Volumenveränderung des Mediums mechanisch eingeschränkt ist. Das Medium enthält ein Trägermaterial, welches beispielsweise ein Polymer sein kann. Eine Wirkung des Trägermaterials besteht darin, die räumliche Verteilung des wenigstens einen Farbstoffs oder den Farbstoff enthaltender Partikel innerhalb des Mediums zu fixieren. Das Medium kann beispielsweise eine Flüssigkeit umfassen, in welcher der wenigstens eine Farbstoff gelöst ist; je nach Ausführungsform kann das Medium weitere Komponenten beinhalten, wie im Weiteren noch dargelegt wird.

Erfindungsgemäß ist im Medium eine Osmolalität gegeben, welche größer ist als eine vorgegebene maximale Probenosmolalität, für welche eine Verwendung des Sensors vorgesehen ist, und ein Elastizitätsmodul des Restriktionsmittels ist wenigstens 100-mal so groß wie ein Elastizitätsmodul des Trägermaterials. Für Proben mit einer Osmolalität größer als diese maximale Probenosmolalität sollte der erfindungsgemäße Sensor nicht eingesetzt werden, da die Messergebnisse in solch einem Fall nicht verlässlich wären.

Durch die Kombination aus der gegebenen Osmolalität im Medium und der mechanischen Einschränkung der Volumenveränderung des Mediums ergibt sich eine definierte Aufnahme von Lösungsmittel, beispielsweise Wasser, in das Medium, wenn der Sensor mit einer Probe in Kontakt gebracht wird, in welcher der Analyt, also der nachzuweisende Stoff, in dem Lösungsmittel gelöst ist. Für gasförmige Proben kann entsprechend Wasser in Form von in der Probe enthaltenem Wasserdampf in den Sensor gelangen, und es ergibt sich analog eine definierte Aufnahme von Wasser. Solange die Osmolalität im Medium größer ist als in der Probe, wirkt der osmotische Druck auf ein Einströmen des Lösungsmittels in das Medium hin. Dieser Zustrom von Lösungsmittel würde zu einem Anschwellen des Mediums führen. Durch das Restriktionsmittel ist eine Volumenveränderung des Mediums, also insbesondere ein Anschwellen, aber scharf begrenzt; dadurch ist auch die Aufnahme von Lösungsmittel in das Medium klar begrenzt. In der Folge ergibt sich für einen gegebenen Sensor eine definierte Aufnahme von Lösungsmittel in das Medium, unabhängig von der Osmolalität der Probe, solange die Osmolalität der Probe geringer als die Osmolalität im Medium ist. Diese definierte Aufnahme von Lösungsmittel kann bei der Kalibrierung des Sensors berücksichtigt werden. Damit wird eine Kalibrierung des Sensors unabhängig von der Osmolalität der Probe, in welcher der Sensor eingesetzt wird, solange die Osmolalität der Probe geringer als die Osmolalität im Medium ist.

In bestimmten Ausführungen ist der wenigstens eine Farbstoff in innerhalb des Trägermaterials befindlichen Hohlräumen enthalten. Diese Hohlräume können dabei vom Trägermaterial selbst ausgebildet sein und den wenigstens einen Farbstoff umschließen. Das Trägermaterial kann auch porös sein, und der wenigstens eine Farbstoff kann sich innerhalb der zumindest teilweise miteinander verbundenen Poren des Trägermaterials befinden. Insbesondere kann der wenigstens eine Farbstoff auch an Poreninnenwänden adsorbiert sein.

In anderen Ausführungsformen sind die Hohlräume von Hüllen umschlossen, welche sich vom Trägermaterial unterscheiden. Insbesondere können die Hüllen aus einem Material bestehen, das vom Trägermaterial verschieden ist. Innerhalb des Trägermaterials können beispielsweise hohle Partikel verteilt sein, deren Inneres den wenigstens einen Farbstoff enthält.

In speziellen Ausführungsformen sind die Hohlräume durch Mizellen gebildet, der wenigstens eine Farbstoff befindet sich im Inneren der Mizellen.

In anderen Ausführungsformen ist der wenigstens eine Farbstoff mit dem Trägermaterial homogen vermischt und innerhalb des Trägermaterials fixiert.

Die mechanische Einschränkung einer Volumenveränderung des Mediums wird in einer Ausführungsform dadurch bewirkt, dass das Medium in einer Vielzahl von Vertiefungen angeordnet wird, welche in einer Trägerplatte ausgebildet sind. Die Trägerplatte kann dabei beispielsweise aus einem Glas oder aus einem Kunststoff, beispielsweise Polykarbonat oder Polyethylenterephthalat (PET), bestehen. Auch Trägerplatten aus Metall, beispielsweise Edelstahl, sind möglich. Um eine deutliche Begrenzung der Volumenveränderung zu erreichen, weist das Material der Trägerplatte einen E-Modul auf, der wenigstens dem 100-fachen des E-Moduls des Trägermaterials entspricht. Vorteilhaft für eine wirksame Begrenzung der Volumenänderung ist es ferner, wenn die Füllhöhe einer Vertiefung mit Trägermaterial mindestens den doppelten Wert eines Durchmessers der Vertiefung aufweist. Die Begrenzung der Volumenänderung kann weiter verbessert werden, wenn das Trägermaterial an der Innenwand der Vertiefung haftet. Ist in Ausgestaltungen, die nicht zur Erfindung gehören, kein Trägermaterial vorhanden, so sollte das Medium vollständig in den Vertiefungen eingekapselt werden; dies kann zum Beispiel durch Deckschichten erfolgen, welche die Vertiefungen verschließen.

In manchen Ausführungen reichen die Vertiefungen vollständig durch die Trägerplatte hindurch, stellen also Löcher in der Trägerplatte dar.

In einer anderen Ausführungsform wird die mechanische Einschränkung einer Volumenveränderung des Mediums dadurch erreicht, dass eine Membran, ein Vlies, ein Geflecht, ein Gewebe oder ein Gitter in das Trägermaterial eingebettet ist und das Restriktionsmittel darstellt. Vorteilhaft haftet dabei das Trägermaterial am Restriktionsmittel. Das Restriktionsmittel besteht aus zugfestem Material, das soll hier bedeuten, dass ein Elastizitätsmodul des Restriktionsmittels wenigstens 100-mal so groß wie ein Elastizitätsmodul des Trägermaterials ist.

In einer anderen Ausführungsform des erfindungsgemäßen Sensors umschließt eine Hülle das Medium teilweise. Das Medium enthält dabei ein Trägermaterial, die Hülle bildet das Restriktionsmittel.

In einer weiteren Ausführungsform wird das Medium vollständig von einer Hülle umschlossen. Auch hier bildet die Hülle das Restriktionsmittel. Im Medium kann ein Trägermaterial vorhanden sein, es können aber auch nicht zur Erfindung gehörige Ausprägungen ohne Trägermaterial eingesetzt werden.

In einer Weiterbildung der vorstehenden Fälle sind durch eine Hülle, welche das Restriktionsmittel darstellt, mehrere Hohlräume gebildet, welche vom Material der Hülle ganz oder teilweise umschlossen werden. In jedem Hohlraum befindet sich ein Medium mit einem Farbstoff. Dabei unterscheiden sich wenigstens zwei der eingesetzten Farbstoffe voneinander. Die Farbstoffe können beispielsweise auf unterschiedliche Analyten reagieren und so jeweils vom Ort des Sensors Informationen zu unterschiedlichen Analyten liefern.

Die das Restriktionsmittel bildende Hülle in den vorstehenden Ausführungsformen muss eine ausreichende mechanische Zugfestigkeit aufweisen, um einer Volumenänderung des Mediums einen hinreichenden mechanischen Widerstand entgegensetzen zu können. Geeignete Materialien für die Hülle sind zum Beispiel Polyvinylidenfluorid(PVDF), Teflon, Polyethersulfon, Polysulfon, Polystyrol, Siliziumdioxid, oder Ormosile; die möglichen Materialien sind jedoch nicht auf die in dieser Auflistung genannten beschränkt.

In einer Ausführungsform des erfindungsgemäßen Sensors ist der wenigstens eine Farbstoff mit einer Pufferlösung vermischt, die dann als Teil des Mediums anzusehen ist. Dies findet vor allem bei jenen Sensoren Anwendung, bei denen die Sensorwirkung auf einem pH-Wert-abhängigen optischen Verhalten des wenigstens einen Farbstoffs beruht, und der Analyt eine pH-Wert-Änderung im Medium bewirkt.

Die Osmolalität im Medium kann bei der Herstellung des Sensors durch Zusatz wenigstens einer Substanz zum Medium eingestellt werden. In Ausführungsformen kann die wenigstens eine Substanz mit dem wenigstens einen Farbstoff vermischt werden. Als Substanzen zur Einstellung der Osmolalität im Medium können beispielsweise Salze, etwa NaCI oder KCl, Polyelektrolyte oder Neutralmoleküle wie zum Beispiel Zucker, etwa Glucose, Fructose, Mannose, Saccharose, verwendet werden. Auch diese zugesetzten Substanzen sind als Teil des Mediums anzusehen. Wichtig ist hierbei selbstverständlich, dass die zugesetzten Substanzen den quantitativen Nachweis des Analyten nicht stören.

In einer speziellen Ausführungsform beinhaltet der Sensor eine hygroskopische Substanz. Solche Ausführungsformen lassen sich auch in gasförmigen Proben, etwa an der Atmosphäre, einsetzen. Die hygroskopische Substanz nimmt aus der Probe Wasser auf, welches in der Probe etwa als Wasserdampf vorliegt. Damit wird für den wenigstens einen Farbstoff eine wässrige Umgebung erzeugt. Es können daher Farbstoffe und Zusätze wie etwa Puffer verwendet werden, welche sonst auf wässrige Proben beschränkt sind, um Messungen in der Gasphase vorzunehmen. Vorzugsweise ist die hygroskopische Substanz mit dem wenigstens einen Farbstoff vermischt.

Das optische Verhalten des Farbstoffs, welches zum quantitativen Nachweis eines Analyten genutzt wird, kann beispielsweise eine Lumineszenz sein, wobei Lumineszenz mindestens Phosphoreszenz und Fluoreszenz umfasst. Ebenso kann eine Lichtreflektion oder Lichtabsorption zum quantitativen Nachweis des Analyten genutzt werden. Eine weitere Möglichkeit ist, eine Farbe des Farbstoffs zu nutzen. Dabei zeigen jeweils die Farbe des Farbstoffs, die Lichtreflektion oder -absorption, oder die Lumineszenz eine Abhängigkeit von dem Analyten. Diese Abhängigkeit kann im Falle einer Lumineszenz darin bestehen, dass eine Relaxationszeit der Lumineszenz, es kann sich hierbei um eine Relaxationszeit für die Intensität der Lumineszenz oder für eine Polarisation der Lumineszenz handeln, von dem Analyten abhängt. Ebenso ist es denkbar, dass Intensität oder Wellenlänge des auftretenden Lumineszenzlichts von dem Analyten abhängen. Im Falle einer Farbe kann der Farbstoff je nach Konzentration oder Partialdruck des Analyten eine andere Farbe annehmen. Im Falle von Lichtreflektion oder -absorption kann sich die Reflektivität beziehungsweise der Absorptionsgrad einer den Farbstoff enthaltenden Schicht für bestimmte Lichtwellenlängen in Abhängigkeit vom Analyten ändern. Es kann auch mehr als eine Art optisches Verhalten für die Messung ausgenutzt werden, etwa eine Relaxationszeit der Lumineszenz und ein Absorptionsverhalten. Zu diesem Zweck kann mehr als ein Farbstoff verwendet werden, so dass etwa das Lumineszenzverhalten eines ersten Farbstoffs und das Absorptionsverhalten eines zweiten Farbstoffs ausgewertet werden, um einen Analyten quantitativ zu bestimmen. Bei Verwendung mehr als eines Farbstoffs kann auch die Effizienz strahlungsloser Energieübertragung zwischen den Farbstoffen, beispielsweise der Förster-Resonanzenergietransfer, soweit diese Effizienz quantitativ vom Analyten abhängt, zur quantitativen Bestimmung des Analyten genutzt werden.

Die Abhängigkeit des optischen Verhaltens vom Analyten kann aus einer direkten Wechselwirkung zwischen dem Analyten und dem wenigstens einen Farbstoff resultieren, etwa einem Energieaustausch oder einer chemischen Reaktion zwischen Molekülen des Farbstoffs und des Analyten, oder aus einer indirekten Wechselwirkung über dem Farbstoff zugesetzte Substanzen. Generelle Voraussetzung für das Funktionieren des Sensors ist damit, dass der Analyt mit dem wenigstens einen Farbstoff in eine solche direkte oder indirekte Wechselwirkung treten kann. Befinden sich bei einem erfindungsgemäßen Sensor beispielsweise Farbstoff und Pufferlösung in Hohlräumen innerhalb eines Trägermaterials, oder sind von dem Restriktionsmittel umschlossen, so muss der Analyt durch das Trägermaterial beziehungsweise das Restriktionsmittel diffundieren können, um das Gemisch aus Farbstoff und Pufferlösung zu erreichen.

Beispiele für Analyten sind Gase in gasförmigen Gemischen oder in Flüssigkeiten gelöste Gase. Beispielsweise kann in Wasser gelöstes Gas, etwa Schwefeldioxid, Kohlendioxid, Kohlenmonoxid, oder Ammoniak durch einen erfindungsgemäßen Sensor erfasst werden. So ist das in Wasser basisch reagierende Ammoniak ein Beispiel, bei dem als Farbstoff für den Sensor ein Farbstoff mit pH-Wert-abhängigem optischen Verhalten gewählt werden kann. In Abhängigkeit von der Konzentration des in der Probe gelösten Ammoniaks stellt sich innerhalb des Mediums ein pH-Wert ein, welcher aus dem optischen Verhalten des Farbstoffs bestimmt werden kann. Indirekt ist so ein Rückschluss auf die Ammoniakkonzentration möglich. Selbstverständlich ist eine direkte Kalibrierung der Ammoniakkonzentration gegen das optische Verhalten möglich, eine tatsächliche Bestimmung eines pH-Wertes ist dann nicht erforderlich. Das eben angeführte Beispiel des Ammoniak-Nachweises ist auch ein Beispiel für eine indirekte Wechselwirkung zwischen dem Farbstoff und dem Analyten, hier also dem Ammoniak. Die Wechselwirkung geschieht hier über eine mit dem Farbstoff vermischte Pufferlösung, indem der Analyt den pH-Wert der Pufferlösung ändert, und das optische Verhalten des Farbstoffs vom pH-Wert seiner Umgebung, hier also der Pufferlösung, abhängt. Analoge Aussagen gelten auch für Schwefeldioxid und weitere Gase. Ein Beispiel für einen Farbstoff mit einem pH-Wert-abhängigen Fluoreszenzverhalten, mit dem Kohlendioxid nachzuweisen ist, ist Hydroxypyrentrisulfonsäure (HPTS). Zur Messung von Schwefeldioxid kann etwa Bromkresolrot verwendet werden. Für den quantitativen Nachweis von Ammoniak kann Bromthymolblau oder Bromphenolblau eingesetzt werden. Zahlreiche weitere Farbstoffe und ihre Einsatzmöglichkeiten für den quantitativen Nachweis unterschiedlichster Stoffe sind dem Fachmann hinreichend bekannt.

Erfindungsgemäße Sensoren können aber auch zum quantitativen Nachweis anderer gelöster Stoffe, auch von Ionen, verwendet werden.

Eine Messanordnung zum quantitativen Nachweis eines Analyten in einer Probe umfasst einen erfindungsgemäßen Sensor wie vorstehend beschrieben. Eine Steuer- und Auswerteeinheit ist vorgesehen, um aus dem optischen Verhalten des wenigstens einen Farbstoffs des Sensors den Analyten quantitativ zu bestimmen. Außerdem umfasst die Messanordnung eine Optik, welche Licht von der Steuer- und Auswerteeinheit zum Sensor führen kann, und ebenso Licht vom Sensor zur Steuer- und Auswerteeinheit, um so das optische Verhalten des wenigstens einen Farbstoffs zu erfassen, aus dem dann der Analyt quantitativ bestimmt wird. Das Licht, welches von der Steuer- und Auswerteeinheit zum Sensor geführt wird, kann, je nach Ausgestaltung des Sensors, Anregungslicht für eine Lumineszenz des wenigstens einen Farbstoffs sein, oder Licht, mit welchem eine Farbe, eine Reflektivität oder ein Absorptionsgrad einer den wenigstens einen Farbstoff enthaltenden Schicht im Sensor bestimmt wird. Die Steuer- und Auswerteeinheit steuert dabei das von ihr ausgesendete Licht, beispielsweise können bestimmte Lichtpulsfolgen oder intensitätsmodulierte Lichtsignale erzeugt werden. In Ausführungsformen der Messanordnung ist die Optik eine Freistrahloptik. In anderen Ausführungsformen der Messanordnung ist die Optik eine Lichtleiteroptik.

Durch die Verwendung eines erfindungsgemäßen Sensors ist es möglich, einen Analyten in einer Probe quantitativ zu bestimmen, ohne verschiedene mögliche Probenosmolalitäten bei Kalibrierung der Messanordnung und bei Messungen mit der Messanordnung aufwändig berücksichtigen zu müssen, denn die Kalibrierung eines erfindungsgemäßen Sensors ist unabhängig von der Probenosmolalität, wie vorstehend ausgeführt. Osmolalitätsquerempfindlichkeiten bestehen somit weder für den Sensor noch für die Messanordnung.

Es ist dabei möglich, dass der Sensor, etwa in Form eines Plättchens, an einem Ende des Lichtleiters sitzt und mit diesem Ende des Lichtleiters in die Probe eingebracht wird. Es ist aber ebenso möglich, dass der Sensor etwa im Inneren eines Probenbehälters an einer Wand des Probenbehälters angebracht wird. Die Wand ist dabei für das Licht, das von der Steuer- und Auswerteeinheit zum Sensor geführt wird und auch für das vom Sensor ausgehende Lumineszenzlicht oder vom Sensor reflektierte oder rückgestreute Licht transparent. Das Licht kann dabei durch eine Freistrahloptik geführt werden. Ebenso kann das Licht durch einen Lichtleiter geführt werden, wobei der Lichtleiter an eine entsprechende Stelle an der Außenseite der Wand geführt wird und dort endet; in diesem Fall tritt also das Licht dort aus dem Lichtleiter aus, durchläuft die für es transparente Wand des Probenbehälters und trifft auf den Sensor. Licht vom Sensor nimmt entsprechend den umgekehrten Weg. Eine weitere Möglichkeit ist, dass ein oder mehrere erfindungsgemäße Sensoren innerhalb einer Probe verteilt sind, beispielsweise in einer flüssigen Probe schwimmen. Eine Erfassung des optischen Verhaltens der Farbstoffe in den Sensoren kann dann zum Beispiel über eine Kamera erfolgen, die in diesem Fall einen Teil der Steuer- und Auswerteeinheit bildet.

Zur quantitativen Bestimmung des Analyten wird, wie bereits erwähnt, vorteilhaft eine Kalibrierung des Sensors genutzt. Zu diesem Zweck können entsprechende Kalibrierdaten für den Sensor in der Steuer- und Auswerteeinheit bereitgestellt, beispielsweise elektronisch abgespeichert, sein.

Es sei noch darauf hingewiesen, dass zur expliziten quantitativen Bestimmung des Analyten aus dem optischen Verhalten des wenigstens einen Farbstoffs über eine entsprechende Kalibrierung diverse Möglichkeiten bekannt sind. So kann etwa eine Relaxationszeit einer Lumineszenz des wenigstens einen Farbstoffs gegen Partialdruck oder Konzentration des Analyten kalibriert werden. Statt die Relaxationszeit selbst zu benutzen, können auch davon abhängige, mitunter experimentell einfacher und unmittelbarer zu bestimmende Größen genutzt werden, wie zum Beispiel Quotienten von Integralen über den Zeitverlauf von Lumineszenzsignalen oder Phasenverschiebungen zwischen einem modulierten Anregungssignal und der Lumineszenzantwort des Farbstoffs. Diese und weitere Möglichkeiten sind im Stand der Technik hinreichend beschrieben, beispielsweise in der deutschen Patentanmeldung DE102011055272A1 und den darin zitierten Schriften.

Im Folgenden wird die Erfindung an Hand von Ausführungsbeispielen und der beigefügten schematischen Zeichnungen noch näher erläutert.
- **Figur 1**: zeigt einen erfindungsgemäßen Sensor, bei dem das Restriktionsmittel durch eine Trägerplatte mit einer Vielzahl an Vertiefungen gebildet ist.
- **Figur 2**: zeigt einen Querschnitt durch eine Vertiefung der in Figur 1 gezeigten Trägerplatte.
- **Figur 3**: zeigt einen Sensor, bei dem das Restriktionsmittel durch ein Geflecht gegeben ist.
- **Figur 4**: zeigt eine Vielzahl von Hohlräumen im Trägermaterial eines erfindungsgemäßen Sensors.
- **Figur 5**: zeigt eine Abwandlung der in Figur 4 gezeigten Konfiguration.
- **Figur 6**: zeigt einen weiteren Querschnitt durch einen Sensor mit Trägerplatte.
- **Figur 7**: zeigt einen Querschnitt durch einen Sensor mit einer vollständig durchbrochenen Trägerplatte.
- **Figur 8**: zeigt einen Sensor, bei dem das Medium vollständig vom Restriktionsmittel umschlossen ist.
- **Figur 9**: zeigt einen Sensor, bei welchem das Medium in mehr als einem Hohlraum angeordnet und vollständig vom Restriktionsmittel umschlossen ist.
- **Figur 10**: zeigt schematisch eine Messanordnung, bei der der erfindungsgemäße Sensor verwendet wird.
- **Figur 11**: zeigt schematisch eine weitere Variante einer Messanordnung, bei der der erfindungsgemäße Sensor verwendet wird.
- **Figur 12**: zeigt schematisch eine weitere Variante einer Messanordnung, bei der der erfindungsgemäße Sensor verwendet wird.

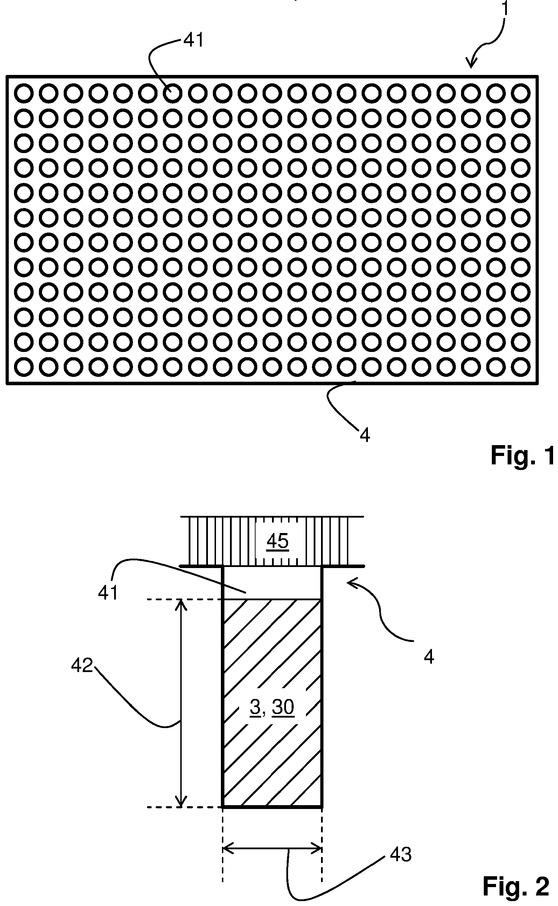

**Figur 1** zeigt eine mögliche Ausführungsform eines erfindungsgemäßen Sensors 1. In einer Trägerplatte 4 sind eine Vielzahl von Vertiefungen 41 ausgebildet. Bei der Herstellung des Sensors 1 kann beispielsweise ein Sensorgemisch aus dem wenigstens einen Farbstoff und einem Medium, welches Pufferlösung und / oder Substanzen, etwa Salze, Polyelektrolyte oder Zucker, zur Einstellung der Osmolalität sowie unpolymerisiertes Trägermaterial enthält, auf die Trägerplatte 4 aufgerakelt werden. Durch anschließendes Aussetzen der Trägerplatte 4 an Vakuum wird das aufgerakelte Sensorgemisch in die Vertiefungen 41 eingesogen. Alternativ kann das aufgerakelte Sensorgemisch beispielsweise auch durch Zentrifugation der mit dem Sensorgemisch versehenen Trägerplatte 4 in die Vertiefungen 41 eingebracht werden. Überschüssiges Sensorgemisch wird entfernt, und das Sensorgemisch in den Vertiefungen wird polymerisiert, etwa thermisch oder photoinduziert.

Die gezeigte rechteckige Form des Sensors 1 stellt keine Beschränkung der Erfindung dar. Es ist aber zur Herstellung von erfindungsgemäßen Sensoren möglich, großflächige Trägerplatten in der vorstehend dargelegten Weise mit einem Sensorgemisch zu versehen, und dann aus der Trägerplatte Sensoren der gewünschten Größe und Form zu sägen, zu schneiden, zu stanzen oder anderweitig abzutrennen. Hierzu können auch Sollbruchstellen in der Trägerplatte vorgesehen sein.

Der gezeigte kreisförmige Querschnitt der Vertiefungen 41 stellt keine Beschränkung der Erfindung dar. Ebenso sind andere Querschnitte möglich, etwa rechteckige oder hexagonale; im hexagonalen Fall ist insbesondere eine Bienenwabenstruktur der Trägerplatte 4 denkbar.

Typische Durchmesser der Vertiefungen 41 betragen 10 bis 500 Mikrometer. Typische Tiefen der Vertiefungen 41 betragen 100 bis 500 Mikrometer. Verwendete Materialstärken für die Trägerplatte liegen dann vorteilhafterweise im Bereich von 100 Mikrometer bis 1 Millimeter. Diese Abmessungen stellen jedoch keine Beschränkung der Erfindung dar. Es sind durchaus auch Durchmesser vom Nanometerbereich bis zum Zentimeterbereich und darüber hinaus denkbar.

**Figur 2** zeigt einen Querschnitt durch eine Vertiefung 41 in einer Trägerplatte 4, wie sie in Figur 1 gezeigt ist. Die Vertiefung 41 ist mit einem Medium 3 gefüllt, welches Trägermaterial 30 und den (hier nicht dargestellten) wenigstens einen Farbstoff enthält. Im gezeigten Beispiel ist eine Füllhöhe 42 der Vertiefung 41 mit dem Medium 3 größer als das Doppelte eines Durchmessers 43 der Vertiefung 41. Wie oben bereits erwähnt, trägt eine solche Dimensionierung zur Beschränkung einer Volumenänderung, insbesondere eines Aufquellens, des Mediums 3 bei.

Ferner dargestellt ist eine optionale Deckschicht 45, welche die Vertiefungen 41 bedeckt. Ist die Deckschicht 45 bei einer Messung einer zu untersuchenden Probe zugewandt, so ist die Deckschicht 45 vorteilhafterweise durchlässig für den Analyten. Das Material der Trägerplatte 4 ist dann vorzugsweise transparent, die Erfassung des optischen Verhaltens des Farbstoffs kann dann durch das Material der Trägerplatte 4 hindurch erfolgen. In diesem Fall kann die Deckschicht 45 reflektierend ausgebildet sein, was die Erfassung des optischen Verhaltens des Farbstoffs begünstigt, vor allem, wenn es sich bei dem optischen Verhalten um eine Lumineszenzerscheinung handelt. Ebenso kann das Material der Trägerplatte 4 durchlässig für den Analyten sein. In diesem Falle wäre die Trägerplatte 4 bei einer Messung der zu untersuchenden Probe zugewendet. Die Deckschicht 45 ist dann vorzugsweise transparent, die Erfassung des optischen Verhaltens des Farbstoffs kann dann durch das Material der Deckschicht 45 hindurch erfolgen.

Eine Möglichkeit, eine reflektierende Deckschicht 45 zu erhalten, besteht darin, eine unpolymerisierte Schicht, die Titandioxidpartikel enthält, auf die Trägerplatte 4 aufzurakeln, und die aufgerakelte Schicht dann zu polymerisieren. Eine nichttransparente Deckschicht 45 kann beispielsweise durch eine Folie aus PVDF oder Teflon gebildet werden. Die Folie kann etwa auf die Trägerplatte 4 aufgeklebt oder thermisch aufgeschweißt werden. Eine derartige Folie ist durchlässig für Gase. Alternativ kann auch die Trägerplatte 4 aus PVDF oder Teflon bestehen, so dass in diesem Fall die Trägerplatte gasdurchlässig ist.

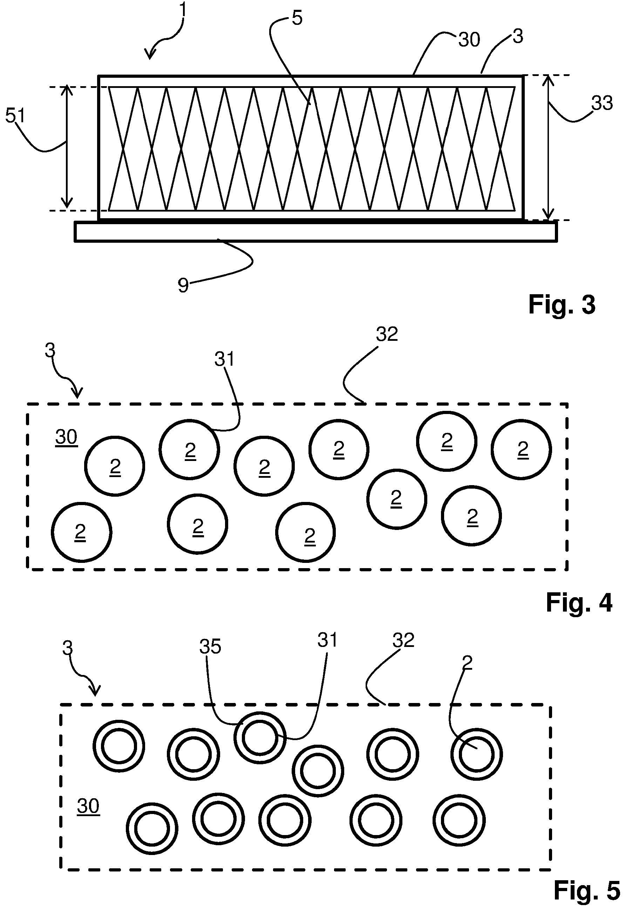

**Figur 3** zeigt eine Ausführungsform des erfindungsgemäßen Sensors 1, bei dem das Restriktionsmittel durch ein Geflecht 5 gegeben ist. Das Geflecht 5 ist in das Trägermaterial 30 eingebettet, welches ein Bestandteil des Mediums 3 ist; das Medium 3 enthält den wenigstens einen Farbstoff und gegebenenfalls weitere Beimischungen, diese sind hier nicht dargestellt. Im gezeigten Beispiel ist das Medium 3 auf einer Stützplatte 9 aufgebracht. Die Stützplatte 9 ist vorzugsweise transparent. Auf diese Weise kann das Medium 3 mit dem wenigstens einen Farbstoff in Kontakt mit einer Probe gebracht werden, und die Erfassung des optischen Verhaltens des Farbstoffs kann durch die transparente Stützplatte 9 hindurch erfolgen. Auch hier kann, analog zur Darstellung in der Figur 2, eine reflektierende Deckschicht vorgesehen werden. Diese wäre in dem diskutierten Beispiel dann parallel zur Stützplatte 9, auf der der Stützplatte 9 gegenüberliegenden Seite des Mediums 3 anzubringen und müsste für den Analyten durchlässig sein. Alternativ kann auch die Stützplatte 9 reflektierend anstatt transparent ausgebildet sein. Die Erfassung des optischen Verhaltens des Farbstoffs würde dann von der der Stützplatte 9 gegenüberliegenden Seite des Sensors 1 aus erfolgen, und die Stützplatte 9 müsste für den Analyten durchlässig sein.

Ein Sensor 1 dieser Art kann etwa hergestellt werden, indem ein Sensorgemisch der schon erwähnten Art auf die Stützplatte 9 aufgerakelt wird, und anschließend das Geflecht 5 auf das Sensorgemisch aufgelegt wird. Das unpolymerisierte Sensorgemisch umschließt das Geflecht 5, so dass letztlich das Geflecht 5 in dem Medium 3, und damit insbesondere im Trägermaterial 30, eingebettet ist. Dann kann das Trägermaterial 30 polymerisiert werden, etwa thermisch oder photoinduziert.

Eine typische Dicke der aufgerakelten Schicht des Sensorgemisches liegt zwischen 10 und 1000 Mikrometer. Eine Dicke 51 des Restriktionsmittels 5 ergibt sich vorteilhaft als die Dicke der aufgerakelten Schicht dividiert durch (1 - Porosität des Restriktionsmittels). Es resultiert eine Gesamtdicke 33 der aufgebrachten Schicht des Mediums 3 mit eingebettetem Restriktionsmittel 5.

Statt eines Geflechts können auch eine Membran, ein Vlies, ein Gewebe oder ein Gitter verwendet werden. Konkret kann auch ein PET-Vlies (z.B. Freudenberg, novatexx 2481) benutzt werden. Im Falle eines Vlieses oder eines Gewebes kann das Sensorgemisch in Vlies oder Gewebe durch Kapillarwirkung eingesaugt werden und dieses imprägnieren. Vorteilhaft sind Vlies oder Gewebe dabei oleophin. Eine Haftung eines polymeren Trägermaterials an dem als Vlies, Gewebe, Geflecht, Gitter oder Membran ausgebildeten Restriktionsmittel kann durch eine Aktivierung des Restriktionsmittels mittels einer Plasmabehandlung, einer Koronabehandlung oder eines Primers verbessert werden.

**Figur 4** zeigt einen Bereich 32 des Mediums 3. Das Medium 3 enthält den wenigstens einen Farbstoff 2 in innerhalb des Trägermaterials 30 ausgebildeten Hohlräumen 31. Außer dem Farbstoff 2 können die Hohlräume 31 je nach Ausführungsform des Sensors 1 auch noch eine Pufferlösung, eine hygroskopische Substanz, eine Substanz zur Einstellung der Osmolalität im Medium 3 oder weitere Substanzen enthalten. Die im Medium 3 eingestellte Osmolalität bezieht sich in der gezeigten Ausführungsform auf die Osmolalität innerhalb der Hohlräume 31.

Ein derart aufgebautes Medium 3 kann sowohl bei Sensoren 1 zum Einsatz kommen, in denen das Medium 3 in Vertiefungen 41 einer Trägerplatte 4 angeordnet ist, so wie in den Figuren 1 und 2 gezeigt, als auch in Sensoren 1 mit in das Medium 3 eingebettetem Restriktionsmittel, wie in Figur 3 gezeigt.

Bei den Hohlräumen 31 kann es sich beispielsweise um Mizellen handeln. Ein derart strukturiertes Trägermaterial 3 kann etwa dadurch erzielt werden, dass ein Mizellen bildendes Gemisch aus Farbstoff, Puffer und gegebenenfalls weiteren Substanzen in einem Silikonmonomer emulgiert wird, das Silikonmonomer in die Vertiefungen 41 einer Trägerplatte 4 eingebracht oder ein Restriktionsmittel in das Silikonmonomer eingebettet wird, und das Silikonmonomer anschließend vernetzt wird.

**Figur 5** gleicht weitgehend der Figur 4. Jedoch sind die Hohlräume 31 der in der Figur 5 gezeigten Konfiguration durch Hüllen 35 umschlossen, welche sich vom Trägermaterial 30 unterscheiden. Beispielsweise können die Hohlräume 31 das Innere von Nanopartikeln sein, welche in dem Trägermaterial 30 immobilisiert sind.

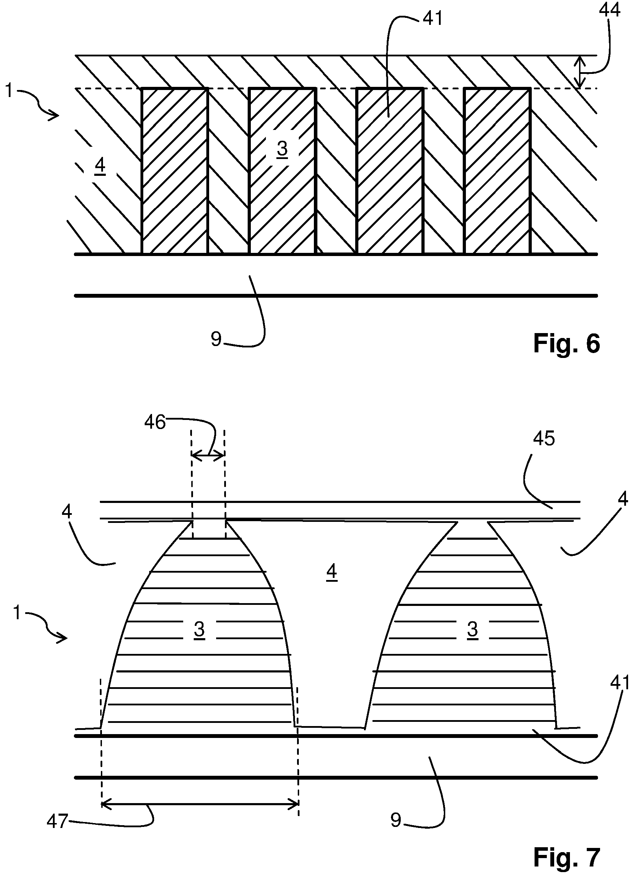

**Figur 6** zeigt einen Querschnitt durch ein weiteres Beispiel eines Sensors mit einer Trägerplatte 4. Die Trägerplatte 4 besteht aus einem für Kohlendioxid durchlässigen Polymer, in welchem durch Prägung Vertiefungen 41 ausgebildet sind. Die Vertiefungen 41 enthalten das Medium 3. Die Trägerplatte 4 wird nach dem Befüllen der Vertiefungen 41 mit dem Medium 3 mit einer transparenten Stützplatte 9 verklebt, so dass die Stützplatte 9 die Öffnungen der Vertiefungen 41 abschließt. Das Medium 3 ist so in den Vertiefungen 41 eingeschlossen. Das Medium 3 kann hier, außerhalb der Erfindung, ohne Trägermaterial verwendet werden, es ist beispielsweise aber auch im Rahmen der Erfindung ein Medium 3 möglich, das wie in der Figur 4 oder der Figur 5 beschrieben aufgebaut ist. In der gezeigten Ausführungsform ist für eine Messung die Stützplatte 9 der Probe abgewandt. Zur Probe hin verbleibt ein Bereich des Polymers der Trägerplatte 4, mit einer Dicke 44, welche etwa zwischen 1 und 50 Mikrometer liegt. Das Material der Trägerplatte kann als optische Isolierung wirken, der Bereich der Dicke 44 kann reflektierend ausgebildet sein.

**Figur 7** zeigt eine Ausführungsform des erfindungsgemäßen Sensors mit Trägerplatte 4, wobei die Trägerplatte 4 durch die Vertiefungen 41 vollständig durchbrochen ist. Die Trägerplatte 4 kann beispielsweise aus Edelstahl (etwa 1.4401, 1.4435, 1.4571) bestehen, in welchem Fall die Vertiefungen 41 zum Beispiel durch Ätzen erzeugt werden können. Die Vertiefungen 41 sind mit dem Medium 3 gefüllt. Die Trägerplatte 4 wird nach dem Befüllen der Vertiefungen 41 mit dem Medium 3 mit einer transparenten Stützplatte 9 verklebt, so dass die Stützplatte 9 Öffnungen der Vertiefungen 41 auf einer Seite der Trägerplatte 4 abschließt. Auf der gegenüberliegenden Seite der Trägerplatte 4 sind die Öffnungen der Vertiefungen 41 durch eine Schicht 45 verschlossen. Die Schicht 45 kann beispielsweise eine Silikonschicht sein, welche Titandioxid enthält und dadurch reflektierend wirkt; auch PVDF ist ein mögliches Material für die Schicht 45. Bei einer Messung wäre die Schicht 45 der Probe zugewandt.

Die Vertiefungen 41 sind in der gezeigten Ausführungsform asymmetrisch in dem Sinne, dass die probenseitigen Öffnungen der Vertiefungen, also die Öffnungen, welche durch die Schicht 45 abgedeckt werden, einen Durchmesser 46 aufweisen, der kleiner ist als ein Durchmesser 47 der Öffnungen, welche durch die Stützplatte 9 bedeckt werden. Es hat sich gezeigt, dass bei einer derartigen asymmetrischen Geometrie die Osmolalitätsquerempfindlichkeit im Vergleich zu einer symmetrischen Geometrie wie in Figur 2 weiter verringert ist, jedoch die Sensoransprechzeit sich im Vergleich verlängert.

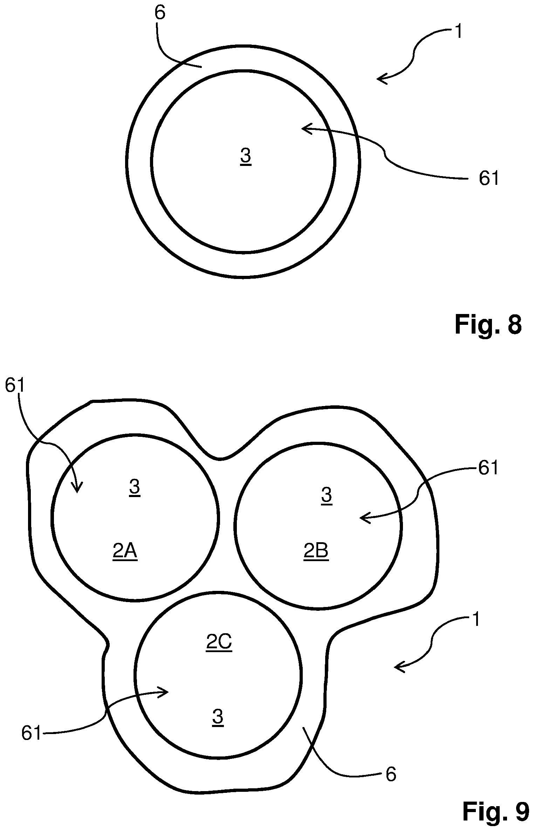

**Figur 8** zeigt einen Sensor 1, bei dem das Medium 3 vollständig von einer Hülle 6 umschlossen ist. Das Medium 3 befindet sich in einem durch die Hülle 6 umschlossenen Hohlraum 61. Die Hülle 6 ist in dieser Ausführungsform das Restriktionsmittel. Die Hülle 6 muss sowohl durchlässig für den Analyten sein als auch transparent für die bei der Erfassung des optischen Verhaltens des wenigstens einen Farbstoffs auftretenden Lichtwellenlängen. Das Medium 3 enthält den nicht gezeigten wenigstens einen Farbstoff, und kann des Weiteren beispielsweise eine Pufferlösung und Substanzen zur Einstellung der Osmolalität des Mediums enthalten. Ein Trägermaterial ist außerhalb der Erfindung nicht erforderlich, die Verwendung eines Trägermaterials ist jedoch möglich, der gezeigte Sensor 1 liegt dann im Umfang der Erfindung. Insbesondere kann das Medium 3 auch wie in der Figur 4 oder Figur 5 gezeigt strukturiert sein. Ein Sensor 1 der hier gezeigten Art kann Kugelform haben, dies stellt jedoch keine Einschränkung der Erfindung dar. Der Durchmesser einer solchen Kugel kann vom Nanometerbereich bis zu beispielsweise 1 Meter oder auch darüber hinaus betragen. Solche Sensoren können beispielsweise in einer Probe schwimmend eingesetzt werden, etwa auch im Inneren eines Bioreaktors oder im Inneren einer Zelle.

Ferner ist es möglich, eine Vielzahl solcher Sensoren 1 in einer umgebenden Matrix, beispielsweise einem Polymer, zu immobilisieren. Die Matrix muss dabei für den Analyten durchlässig sein.

**Figur 9** zeigt ein weiteres Beispiel eines Sensors 1. Wie in der Ausführungsform der Figur 8 bildet eine Hülle 6 das Restriktionsmittel. Die Hülle 6 umschließt in dieser Ausführungsform drei Hohlräume 61, in welchen sich jeweils ein Medium 3 befindet. In jedem Hohlraum 61 befindet sich jeweils ein Farbstoff, 2A, 2B und 2C. Dabei sind in der gezeigten Ausführungsform die Farbstoffe 2A, 2B und 2C voneinander verschieden. Entsprechend können sich auch die Medien 3 in den einzelnen Hohlräumen 61 voneinander unterscheiden, beispielsweise um an den jeweiligen Farbstoff 2A, 2B oder 2C angepasst zu sein. Es sind ebenso Ausführungsformen mit nur zwei Hohlräumen 61 oder solche mit mehr als drei Hohlräumen 61 denkbar.

Zeigt das optische Verhalten der Farbstoffe 2A, 2B, 2C eine Abhängigkeit von einem jeweils anderen Analyten, so kann ein Sensor der gezeigten Art verwendet werden, um Informationen über diese Analyten vom, mit einer durch die Sensorabmessungen gegebenen Genauigkeit, selben Ort innerhalb einer Probe zu erhalten.

Außer den in den Figuren 8 und 9 gezeigten Sensoren 1, bei denen das Restriktionsmittel das Medium 3 als Hülle 6 jeweils vollständig umschließt, sind auch Sensoren denkbar, bei denen das Medium 3, das in diesem Fall vorteilhaft ein Trägermaterial umfasst, nur teilweise vom Restriktionsmittel umschlossen sind.

Ein Sensor wie in der Figur 9 gezeigt, kann auch erhalten werden, indem mehrere, hier konkret drei, Sensoren der in Figur 8 gezeigten Art mit ihren Hüllen 6 verbunden, etwa verklebt oder verschweißt werden.

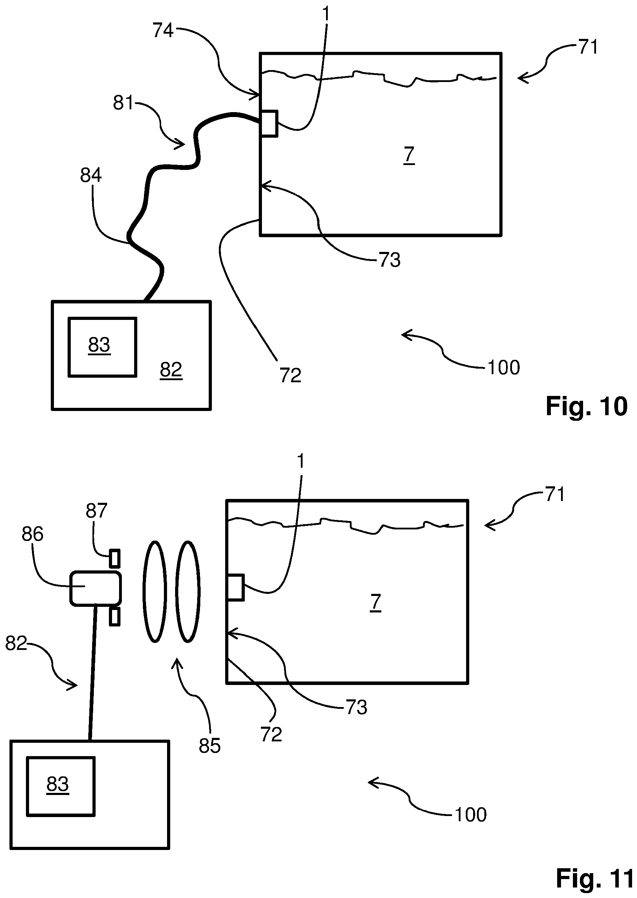

**Figur 10** zeigt eine Messanordnung 100, bei der ein erfindungsgemäßer Sensor 1 verwendet wird. Der Sensor 1 befindet sich innerhalb eines Probenbehälters 71 in Kontakt mit einer wässrigen Probe 7, und ist an einer Innenseite 73 einer Wand 72 des Probenbehälters 71 fixiert. Im gezeigten Beispiel ist das vom Analyten abhängige optische Verhalten des Farbstoffs des Sensors 1 ein Lumineszenzverhalten. Die Optik 81 zur Erfassung des Lumineszenzverhaltens umfasst einen Lichtleiter 84. Über den Lichtleiter 84 kann Anregungslicht zu dem Sensor 1 geführt werden, um die Lumineszenz des Farbstoffs anzuregen. Ebenso kann Lumineszenzlicht von dem Farbstoff über den Lichtleiter 84 zu der Steuer- und Auswerteeinrichtung 82 geführt werden, welche aus dem empfangenen Lumineszenzlicht eine Konzentration oder einen Partialdruck des Analyten in der Probe 7 ermittelt. Hierbei wird auf bereitgestellte Kalibrierdaten 83 für den Sensor 1 zurückgegriffen. Der Lichtleiter 84 endet im gezeigten Beispiel an einer Außenseite 74 der Wand 72 des Probenbehälters 71. Die Wand 72 des Probenbehälters 71 ist hierbei für das Anregungslicht wie für das Lumineszenzlicht transparent. Zur Optik 81 gehören zumindest noch optische Einkoppel- und Auskoppelelemente um Licht in den Lichtleiter 84 ein- oder auszuleiten. Solche Einkoppel- und Auskoppelelemente sind dem Fachmann hinlänglich bekannt und hier nicht dargestellt.

**Figur 11** entspricht weitgehend der Figur 10, jedoch wird als Optik hier eine Freistrahloptik 85 verwendet. Ferner gezeigt ist ein Detektor 86 für Lumineszenzlicht und ein Ringlicht 87 als Lichtquelle zur Anregung der Lumineszenz, welche zur Steuer- und Auswerteeinheit 82 gehören. Die übrigen Elemente wurden bereits in Figur 10 beschrieben. Die Darstellung der Freistrahloptik 85 ist lediglich schematisch, die Freistrahloptik 85 kann neben Linsen auch weitere optische Elemente wie Filter und Blenden enthalten.

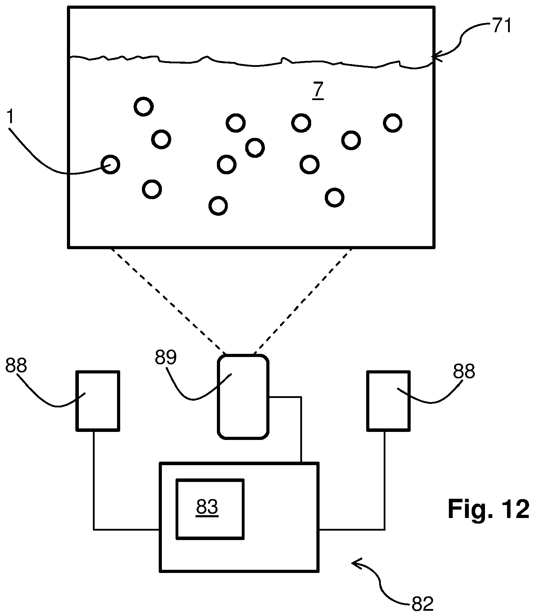

**Figur 12** zeigt eine Probe 7 in einem Probenbehälter 71. Eine Vielzahl von erfindungsgemäßen Sensoren 1 der in Figur 8 oder Figur 9 gezeigten Ausführungsform schwimmen in der flüssigen Probe 7. Zur Erfassung des optischen Verhaltens des in den Sensoren 1 enthaltenen wenigstens einen Farbstoffs sind der Steuer- und Auswerteeinheit 82 zugehörige Lichtquellen 88 und eine ebenfalls der Steuer- und Auswerteeinheit 82 zugehörige Kamera 89 vorgesehen. Die Steuer- und Auswerteeinheit 82 wertet mit der Kamera 89 aufgenommene Bilder aus und kann dadurch eine quantitative Bestimmung des Analyten ortsaufgelöst durchführen.

## Patentansprüche

1. Sensor (1) zum quantitativen Nachweis eines Analyten in einer Probe (7), wobei der Sensor (1) zumindest ein Medium (3) und ein Restriktionsmittel (4, 5, 6), durch welches eine Volumenveränderung des Mediums (3) mechanisch eingeschränkt ist, umfasst, wobei das Medium (3) wenigstens einen Farbstoff (2), der ein optisches Verhalten aufweist, welches innerhalb des Sensors (1) durch den Analyten beeinflussbar ist, und ein Trägermaterial (30) enthält, **dadurch gekennzeichnet, dass**
eine Osmolalität im Medium (3) gegeben ist, welche größer ist als eine vorgegebene maximale Probenosmolalität, für die der Sensor (1) vorgesehen ist, und
ein Elastizitätsmodul des Restriktionsmittels (4, 5, 6) wenigstens 100-mal so groß wie ein Elastizitätsmodul des Trägermaterials (30) ist.

2. Sensor (1) nach Anspruch 1, **wobei** das Trägermaterial (30) ein Polymer ist.

3. Sensor (1) nach einem der Ansprüche 1 oder 2, **wobei** der Farbstoff (2) in innerhalb des Trägermaterials (30) befindlichen Hohlräumen (31) enthalten ist.

4. Sensor (1) nach Anspruch 3, **wobei** die Hohlräume (31) von Hüllen (35) umschlossen sind, die sich vom Trägermaterial (30) unterscheiden.

5. Sensor (1) nach Anspruch 3, **wobei** die Hohlräume (31) durch Mizellen gebildet sind.

6. Sensor (1) nach Anspruch 1 oder 2, **wobei** der Farbstoff (2) mit dem Trägermaterial (30) homogen vermischt ist.

7. Sensor (1) nach einem der Ansprüche 1 bis 6, **wobei** das Restriktionsmittel eine Trägerplatte (4) mit einer Vielzahl von Vertiefungen (41) ist, in welchen das Medium (3) angeordnet ist.

8. Sensor (1) nach Anspruch 7, **wobei** eine Füllhöhe (42) einer Vertiefung (41) mit dem Medium (3) mindestens den doppelten Wert eines Durchmessers (43) der Vertiefung (41) aufweist.

9. Sensor (1) nach Anspruch 7 oder 8, **wobei** mindestens eine Vertiefung (41) die Trägerplatte (4) durchbricht.

10. Sensor (1) nach einem der Ansprüche 1 bis 6, **wobei** das Restriktionsmittel in das Trägermaterial (30) eingebettet ist, und das Restriktionsmittel eine Membran, ein Vlies, ein Geflecht (5), ein Gewebe oder ein Gitter ist.

11. Sensor (1) nach einem der Ansprüche 1 bis 6, **wobei** das Restriktionsmittel eine Hülle (6) ist, welche das Medium (3) teilweise umschließt.

12. Sensor (1) nach einem der Ansprüche 1 bis 6, **wobei** das Restriktionsmittel eine Hülle (6) ist, welche das Medium (3) vollständig umschließt.

13. Sensor (1) nach einem der Ansprüche 11 oder 12 mit mehreren durch die Hülle (6) gebildeten Hohlräumen (61), in welchen sich jeweils ein Farbstoff (2A, 2B, 2C) befindet, **wobei** sich mindestens zwei der Farbstoffe (2A, 2B, 2C) voneinander unterscheiden.

14. Sensor (1) nach einem der vorstehenden Ansprüche, **wobei** der wenigstens eine Farbstoff (2) mit einer Pufferlösung vermischt ist.

15. Sensor (1) nach einem der vorstehenden Ansprüche, **wobei** die Osmolalität im Medium (3) durch Zusatz wenigstens einer Substanz zum Medium (3) eingestellt ist.

16. Sensor (1) nach einem der vorstehenden Ansprüche, **wobei** der Sensor (1) eine hygroskopische Substanz beinhaltet.

17. Sensor (1) nach einem der vorstehenden Ansprüche, **wobei** das optische Verhalten des wenigstens einen Farbstoffs (2) mindestens eine Lumineszenz oder eine Farbe oder eine Lichtreflektion oder eine Lichtabsorption oder eine Polarisation beinhaltet.

## Claims

1. Sensor (1) for quantitatively detecting an analyte in a sample (7), wherein the sensor (1) comprises at least one medium (3) and a restriction means (4, 5, 6) by means of which a change in volume of the medium (3) is mechanically restricted, wherein the medium (3) contains at least one dye (2) having an optical behavior which can be influenced within the sensor (1) by the analyte and wherein the medium (3) contains a carrier material (30),
**characterized in that**
an osmolality in the medium (3) is given which is greater than a predetermined maximum sample osmolality for which the sensor (1) is provided, and
a modulus of elasticity of the restriction means (4, 5, 6) is at least 100 times as great as a modulus of elasticity of the carrier material (30).

2. Sensor (1) according to claim 1, wherein the carrier material (30) is a polymer.

3. Sensor (1) according to one of claims 1 or 2, wherein the dye (2) is contained in cavities (31) located within the carrier material (30).

4. Sensor (1) according to claim 3, wherein the cavities (31) are enclosed by sheaths (35) that are different from the carrier material (30).

5. Sensor (1) according to claim 3, wherein the cavities (31) are formed by micelles.

6. Sensor (1) according to claim 1 or 2, wherein the dye (2) is homogeneously mixed with the carrier material (30).

7. Sensor (1) according to one of claims 1 to 6, wherein the restriction means is a carrier plate (4) having a plurality of depressions (41) in which the medium (3) is arranged.

8. Sensor (1) according to claim 7, wherein a filling height (42) of a depression (41) with the medium (3) has at least twice the value of a diameter (43) of the depression (41).

9. Sensor (1) according to claim 7 or 8, wherein at least one depression (41) breaks through the carrier plate (4).

10. Sensor (1) according to one of claims 1 to 6, wherein the restriction means is embedded in the carrier material (30), and the restriction means is a membrane, a nonwoven material, a braid (5), a woven fabric or a mesh.

11. Sensor (1) according to one of claims 1 to 6, wherein the restriction means is a sheath (6) which partially encloses the medium (3).

12. Sensor (1) according to one of claims 1 to 6, wherein the restriction means is a sheath (6) which completely encloses the medium (3).

13. Sensor (1) according to one of claims 11 or 12, having a plurality of cavities (61) formed by the sheath (6), in each of which cavities (61) a dye (2A, 2B, 2C) is located, wherein at least two of the dyes (2A, 2B, 2C) are different from one another.

14. Sensor (1) according to one of the preceding claims, wherein the at least one dye (2) is mixed with a buffer solution.

15. Sensor (1) according to one of the preceding claims, wherein the osmolality in the medium (3) is adjusted by adding at least one substance to the medium (3).

16. Sensor (1) according to one of the preceding claims, wherein the sensor (1) comprises a hygroscopic substance.

17. Sensor (1) according to one of the preceding claims, wherein the optical behavior of the at least one dye (2) contains at least a luminescence or a color or a light reflection or a light absorption or a polarization.

## Revendications

1. Capteur (1) pour la détermination quantitative d'un analyte dans un échantillon (7), le capteur (1) comprenant au moins un milieu (3) et un moyen de restriction (4, 5, 6) par lequel un changement de volume du milieu (3) est limité mécaniquement, le milieu (3) contenant au moins un colorant (2), qui présente un comportement optique qui peut être influencé par l'analyte à l'intérieur du capteur (1), ainsi qu'un matériau de support (30),
**caractérisé en ce que**
il existe dans le milieu (3) une osmolalité qui est supérieure à une osmolalité d'échantillon maximale prédéfinie pour laquelle le capteur (1) est prévu, et
un module d'élasticité du moyen de restriction (4, 5, 6) est au moins 100 fois plus grand qu'un module d'élasticité du matériau de support (30).

2. Capteur (1) selon la revendication 1, dans lequel le matériau de support (30) est un polymère.

3. Capteur (1) selon l'une des revendications 1 ou 2, dans lequel le colorant (2) est contenu dans des cavités (31) se trouvant à l'intérieur du matériau de support (30).

4. Capteur (1) selon la revendication 3, dans lequel les cavités (31) sont entourées par des enveloppes (35) qui sont distinctes du matériau de support (30).

5. Capteur (1) selon la revendication 3, dans lequel les cavités (31) sont formées par des micelles.

6. Capteur (1) selon la revendication 1 ou 2, dans lequel le colorant (2) est mélangé de manière homogène avec le matériau de support (30).

7. Capteur (1) selon l'une des revendications 1 à 6, dans lequel le moyen de restriction est une plaque de support (4) comportant une pluralité de dépressions (41) dans lesquelles le milieu (3) est disposé.

8. Capteur (1) selon la revendication 7, dans lequel une hauteur de remplissage (42) d'une dépression (41) avec le milieu (3) présente au moins deux fois la valeur d'un diamètre (43) de la dépression (41).

9. Capteur (1) selon la revendication 7 ou 8, dans lequel au moins une dépression (41) traverse la plaque de support (4).

10. Capteur (1) selon l'une des revendications 1 à 6, dans lequel le moyen de restriction est incorporé dans le matériau de support (30) et le moyen de restriction est une membrane, un non-tissé, un treillis (5), un tissu ou une grille.

11. Capteur (1) selon l'une des revendications 1 à 6, dans lequel le moyen de restriction est une enveloppe (6) qui entoure partiellement le milieu (3).

12. Capteur (1) selon l'une des revendications 1 à 6, dans lequel le moyen de restriction est une enveloppe (6) qui entoure complètement le milieu (3).

13. Capteur (1) selon l'une des revendications 11 ou 12, comportant plusieurs cavités (61) formées par l'enveloppe (6), dans chacune desquelles se trouve un colorant (2A, 2B, 2C), au moins deux des colorants (2A, 2B, 2C) étant différents les uns des autres.

14. Capteur (1) selon l'une des revendications précédentes, dans lequel ledit au moins un colorant (2) est mélangé avec une solution tampon.

15. Capteur (1) selon l'une des revendications précédentes, dans lequel l'osmolalité dans le milieu (3) est réglée par l'ajout d'au moins une substance au milieu (3).

16. Capteur (1) selon l'une des revendications précédentes, dans lequel le capteur (1) contient une substance hygroscopique.

17. Capteur (1) selon l'une des revendications précédentes, dans lequel le comportement optique dudit au moins un colorant (2) comprend au moins une luminescence, une couleur, une réflexion de la lumière, une absorption de la lumière ou une polarisation.
